# EUROPEAN PATENT APPLICATION

(11) **EP 4 481 752 A1**
(43) Date of publication of application: **25.12.2024**
(21) Application number: 23180935.1
(22) Date of filing: 22.06.2023
(51) Int. Cl.: G16H 10/60, G16H 50/20, G16H 50/30, G16H 50/70

(54) **HEALTH DATA IN MEDICAL DASHBOARDS**

(71) Applicant: Koninklijke Philips N.V., 5656 AG Eindhoven (NL)
(72) Inventor: SPELT, Hanne Adriana Alijda, Eindhoven (NL); VAN EE, Raymond, Eindhoven (NL); DENISSEN, Adrianus Johannes Maria, 5656AG Eindhoven (NL); VAN DOOREN, Marieke, Eindhoven (NL); KLAMING, Laura, Eindhoven (NL)
(74) Representative: Philips Intellectual Property & Standards

(57) **Abstract**

A method for ordering health data of a subject to be displayed to a user, where the health data comprises subject data for a plurality of data types. The method comprises determining one or more orders of importance for the data types based on historical data for each data type.

## Description

### FIELD OF THE INVENTION

The invention relates to the field of health data on medical dashboards. In particular, the invention relates to ordering health data for display on the medical dashboards.

### BACKGROUND OF THE INVENTION

Medical specialists (e.g., neurologists) responsible for establishing diagnoses and treatment trajectories typically deal with an overload of information from different sources (e.g., radiology, GP, etc.). This can increase the risk of important information being missed.

Thus, there is a need to reduce the information overload on the medical specialists whilst ensuring they are provided with the important information.

### SUMMARY OF THE INVENTION

The invention is defined by the claims.

According to examples in accordance with an aspect of the invention, there is provided a computer-implemented method for ordering health data of a subject to be displayed to a user, the health data comprising subject data for a plurality of data types, the method comprising determining one or more orders of importance for the data types based on historical data for each data type.

The method may further comprise extracting an importance for each data type using the historical data, wherein the one or more orders of importance are based on the importance for each data type. Extracting may comprise comparing the subject data to the historical data for each data type.

Ordering the data types in terms of their importance can reduce information overload to the user as they can focus on the most important subject data. This reduces the time needed to check the subject's health data and reduces the time taken in decision making as the order of importance can provide the most relevant data directly. Additionally, there is a decreased risk of missing any important information.

An order of importance provides a relative importance between the data types.

The health data provides data indicative of the health of the subject.

The method may further comprise displaying the subject data, for each data type, based on one or more of the orders of importance.

Determining one of the orders of importance may comprise determining differences between the subject data, for each data type, and historical norm values for each corresponding data type, wherein the ordering of the data types is based on the determined differences.

Historical norm values for a data type can be obtained, for example, by measuring the values of the data types on healthy subjects. Historical norm values are known for most data types (e.g., healthy heart rate per age group, weight per age group, blood pressure etc.).

Determining one of the orders of importance may comprise comparing the subject data, for each data type, to historical subject data of the subject, for each corresponding data type, to determine subject-specific differences between the subject data and the historical subject data for each data type, wherein the ordering of the data types is based on the determined subject-specific differences.

The historical subject data can be obtained from longitudinal subject-specific health data. For example, for longer-term conditions, historical subject data for the data types will be available.

Determining one of the orders of importance may be based on a condition-specific order for a suspected clinical condition of the subject, wherein the condition-specific order is based on a historical importance of each data type for the suspected clinical condition.

The importance of the data types can depend based on the suspected clinical condition of the subject. The importance can be pre-determined from literature (e.g., WHO information on incidence and prevalence), existing databases and/or the opinion of experts on the suspected clinical condition.

Determining one of the orders of importance may be based on a user-specific order for the user requesting the subject data to be displayed, wherein the user-specific order is based on the user's historical preferences for displaying the data types.

The user's historical preferences for displaying the data types can include preferences on which data types are displayed as well as preferences in the position of the presentation of the data (e.g., it could be assumed that data types typically positioned at the top and/or at the right of the display are more important to the user). Other examples include different font sizes, display of graphs vs tables etc.

Determining one of the orders of importance may be based on a role-specific order corresponding to the role of the user requesting the subject data to be displayed, wherein the role-specific order is based on historical preferences for displaying the data types for users of the same role.

The method may comprise determining a plurality of orders of importance and further comprise comparing the data types available in the health data of the subject to the data types available in the condition-specific, user-specific and/or role-specific orders and identifying missing data types in the health data.

It is possible that subject data is not available for an important data type. Missing data types can be identified by comparing the available data types in the health data to the data types considered important in the condition/user/role-specific orders. This enables the user to check they are not missing any important data types.

The method may comprise determining a plurality of orders of importance and further comprise comparing the determined orders of importance to each other and identifying a most relevant order of importance for a suspected clinical condition of the subject.

The method may further comprise comparing the determined orders of importance to each other and determining a treatment recommendation for the subject.

The invention also provides a computer program carrier comprising computer program code which, when executed on a computer, causes the computer to perform all of the steps according to the aforementioned method.

The computer program carrier may comprise computer memory (e.g., random-access memory), computer storage (e.g., hard drives, solid-state drives etc.). The computer program carrier may be a bitstream carrying the computer program code.

The invention also provides a system for ordering health data of a subject to be displayed to a user, the health data comprising subject data for a plurality of data types, the system comprising a processor configured to determine one or more orders of importance for the data types based on historical data for each data type.

The processor may be further configured to display the subject data, for each data type, based on one or more of the orders of importance.

The processor may be configured to determine one of the orders of importance by determining differences between the subject data, for each data type, and historical norm values for each corresponding data type, wherein the ordering of the data types is based on the determined differences.

The processor may be configured to determine one of the orders of importance by comparing the subject data, for each data type, to historical subject data of the subject, for each corresponding data type, to determine subject-specific differences between the subject data and the historical subject data for each data type, wherein the ordering of the data types is based on the determined subject-specific differences.

The processor may be configured to determine one of the orders of importance based on a condition-specific order for a suspected clinical condition of the subject, wherein the condition-specific order is based on a historical importance of each data type for the suspected clinical condition.

The processor may be configured to determine one of the orders of importance based on a user-specific order for the user requesting the subject data to be displayed, wherein the user-specific order is based on the user's historical preferences for displaying the data types.

The processor may be configured to determine one of the orders of importance based on a role-specific order corresponding to the role of the user requesting the subject data to be displayed, wherein the role-specific order is based on historical preferences for displaying the data types for users of the same role.

The processor may be configured to determine a plurality of orders of importance and further configured to compare the data types available in the health data of the subject to the data types available in the condition-specific, user-specific and/or role-specific orders and identifying missing data types in the health data.

The processor may be configured to determine a plurality of orders of importance and further configured to compare the determined orders of importance to each other and identifying a most relevant order of importance for a suspected clinical condition of the subject.

The processor may be further configured to compare the determined orders of importance to each other and determining a treatment recommendation for the subject.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
- Fig. 1: shows the generation of a first order of importance;
- Fig. 2: shows the generation of a second order of importance;
- Fig. 3: shows the generation of a third order of importance;
- Fig. 4: shows the generation of a fourth order of importance;
- Fig. 5: shows the generation of a fifth order of importance; and
- Fig. 6: shows the generation of a recommended ordering.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for ordering health data of a subject to be displayed to a user, where the health data comprises subject data for a plurality of data types. The method comprises determining one or more orders of importance for the data types based on historical data for each data type.

In an embodiment, a personalized data-driven recommender system is used toto order medical information. In an example, the system uses a minimum of five orderings as described below. The weights of the orderings may be dynamic and are subject to the output of the system. This enables the ordering of health data based on their value in a meaningful dashboard for a specific subject.

It has been realized that considering historical data corresponding to, for example, the role of the user, the suspected diagnosis of the subject and the point in the treatment trajectory provides meaning to the data related to usage of a medical dashboard. This enables personalization of the medical dashboard to the user, and thereby optimizing of the workflow.

The system may be configured to obtain health data corresponding to a subject, a suspected diagnosis, the health care role of the user and/or user dashboard usage data. The system may comprise a processor running a first algorithm that receives the afore-mentioned data obtained by the system, orders the health data based on potential orderings, weights each ordering based on an importance and provides a personalized recommendation for information ordering. A device (e.g., a medical dashboard) can be used to present the health data to the user.

A second algorithm can be used to compare the personalized recommendation to a database of role-specific information ordering recommendations. Based on the comparison, any missing or unused important data types can be flagged, and workflow recommendations can be provided based on the missing/unused important data types. Based on a database of role-specific recommendations, insights into decision-making could also be provided.

The invention provides a reduction of information overload by providing the health data ordered based on historic relevance/importance. This can be achieved by extracting an importance, for each available data type, from the historical data. In particular, the historical data includes a previous ordering of the data types in the health data.

Workflow optimization can also be achieved by automatically scheduling tests when important information is missing or recommending to not do unnecessary tests when these appear to be less important.

A decreased risk of missing important information can also be achieved by comparing the available data types to historical data corresponding to role-specific dashboards.

Insights in decision making can also be provided to the user by analyzing the weight of each ordering of importance and thereby potentially leading to treatment recommendations.

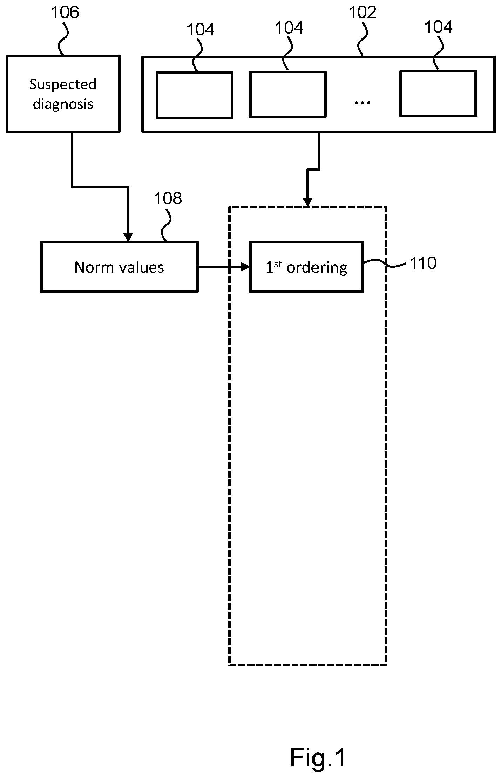

Fig. 1 shows the generation of a first order of importance 110. The subject's health data 102 contains subject data 104 for a plurality of data types. A suspected diagnosis 106 for the subject is also available.

The first order of importance 110 described here is based on deviations from historical norm values 108 for the suspected diagnosis. The data types in the subject's health data may include, for example, a radiology report, blood test results, anamnesis, data from wearables, socioeconomic status and demographic information. Often, the available subject data results from a protocol following the suspected diagnosis 106.

Historical norm values 108 for the different data types considered important for the suspected diagnosis 106 can be formulated based on literature, an existing subject database (e.g., within that hospital), and/or the opinion of an expert. The subject data 104 can be compared with the historical norm values 108 and rank the importance based on the size of the deviation from the norm 108 to arrive at the first order of importance 110. In other words, the data type that deviates most from the norm 108 is ranked highest and placed in the most prominent position in the first order of importance 110.

A dashboard with ordered information (i.e., according to an order of importance) can inform or further sharpen the first broad diagnosis. Based on the test results, new tests can be set up to arrive at a clear analysis. The current diagnosis is indicative of the choice of the following tests and may be output.

It will be understood that the first order of importance 110 can be applied to any setting in which a medical professional uses a dashboard to review patient-specific health data for diagnosis and treatment.

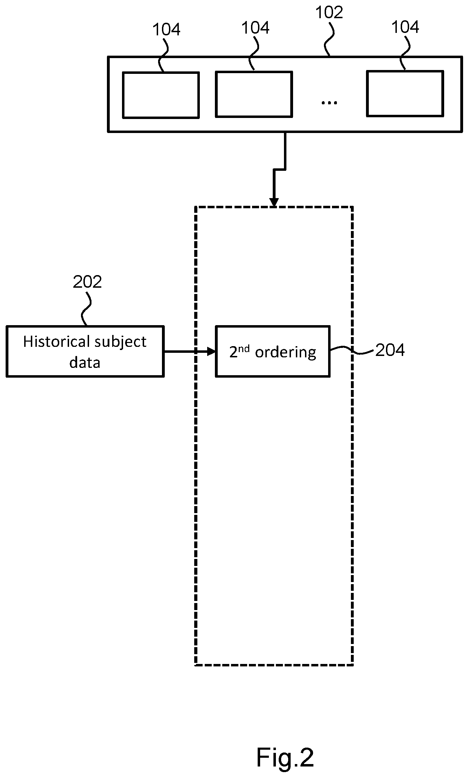

Fig. 2 shows the generation of a second order of importance 204. The second order of importance 204 is based on deviations in longitudinal (i.e., historic) subject data 202. In some cases, there exists historical health data for a subject for example, if similar tests have been performed previously or if the subject has been under treatment for a longer period of time. Thus, there may be a database of historical subject data for the relevant data types to be ordered. The new subject data 104 to can be compared to the historical subject data 202 and the data types can be ranked based on the (normalized) size of the difference between the two measurement moments. That is, the data type that deviates most from historic subject data measurements is placed in a more prominent position on the second ordering 204.

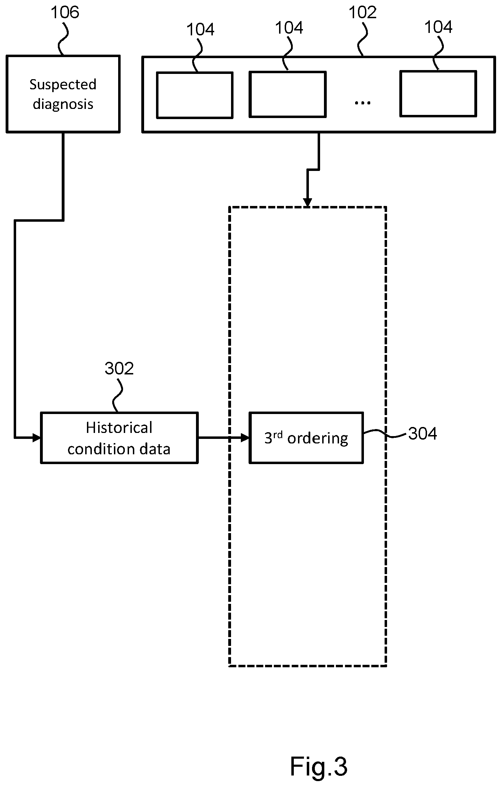

Fig. 3 shows the generation of a third order of importance 304. The third order of importance 304 is based on condition-specific knowledge. In many cases, a suspected diagnosis 106 has already been made (e.g., by the GP) before or during the process that the subject undergoes to arrive at an official diagnosis and treatment. The historical condition data 302 can be derived, for the suspected diagnosis 106, from literature (e.g., world health organization (WHO) information on incidence and prevalence), an existing patient database (e.g., within that hospital), and/or the opinion of an expert.

The data types of the available subject data 104 can be ordered based on historical condition data 302 the suspected diagnosis 106. The third order of importance 304 is based on the importance of a data type for that suspected diagnosis. That is, the most meaningful data type is ranked highest and placed in the most prominent position on the third ordering. Using the historical condition data 302, it can be determined whether the most valuable data type for that diagnosis is part of the health data 102 of the subject. Thus, any missing or unused important data types can be flagged to the user and a workflow adaptation such as automatic scheduling of a test can be output to the user.

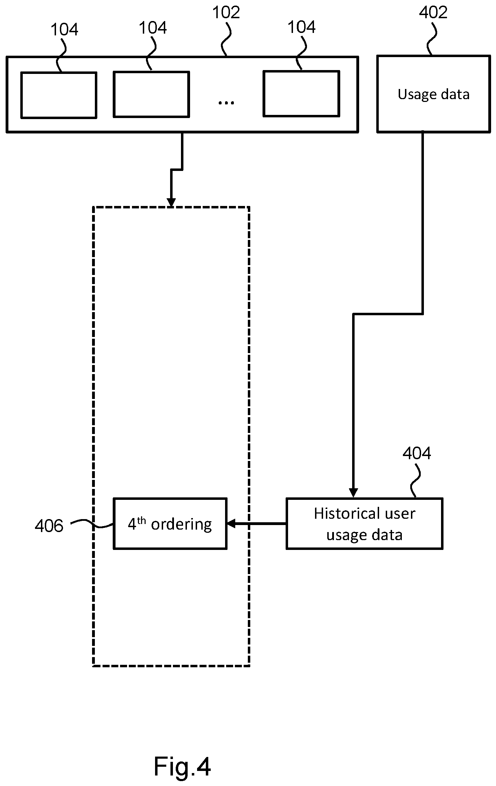

Fig. 4 shows the generation of a fourth order of importance 406. The fourth order of importance 406 is based on user-specific dashboard usage. In this case, the historical user usage data 404 corresponding to the dashboard usage of that user (e.g., click through data, explicit ranking by different doctors, fixations and gaze points, heatmaps, areas of interest, dwell time, revisits, etc.) is used. The historic user usage data 404 can be obtained from a database containing usage data 402 of the dashboard. With prolonged use of the dashboard, a database including the historic user data 404 can be created. User preferences can be extracted from the historical user usage data 404 and the data types can be ranked for the fourth order of importance 406 based on which data usually gets the most attention for a particular user.

The historical user usage data 404 can be provided in a per-condition basis or for any condition. In the case where separate historical user usage data 404 is provided for different conditions, the fourth order of importance 406 is further based on the suspected diagnosis for the subject as this will inform which historical user usage data to use.

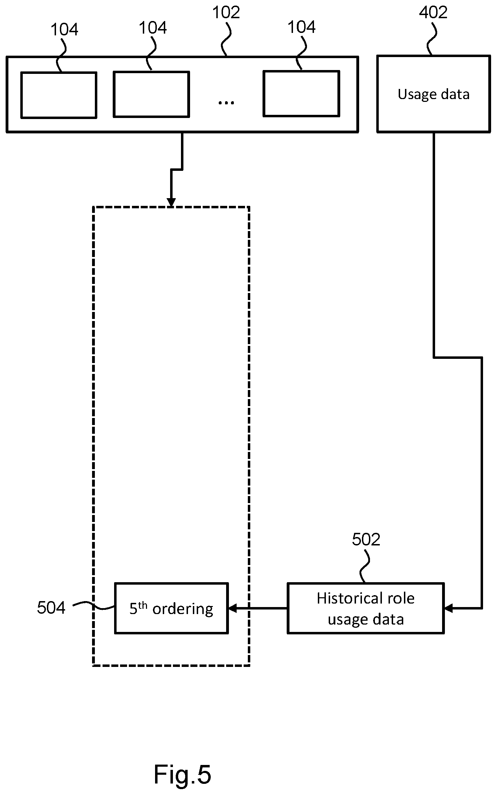

Fig. 5 shows the generation of a fifth order of importance 504. The fifth order of importance is based on role-specific dashboard usage. In this case, it can be assumed that the dashboard is used by a multitude of users with different roles. Thus, historical role usage data 502 can be created with the average usage data for potential roles (e.g., nurse, radiologist, neurologist, etc.) from a database containing usage data 402 of the dashboard.

The data types can thus be ranked based on what people within that specific role would generally prefer. In other words, the data type that is used more often by a particular role is placed in a more prominent position in the fifth order of importance 504 for users with a similar role. The user's preferences with the preferences of most people in that role (i.e., data base with role-specific recommendations) and can flag any missing or unused important data and suggest a workflow adaptation such as automatic scheduling of a test (i.e., output).

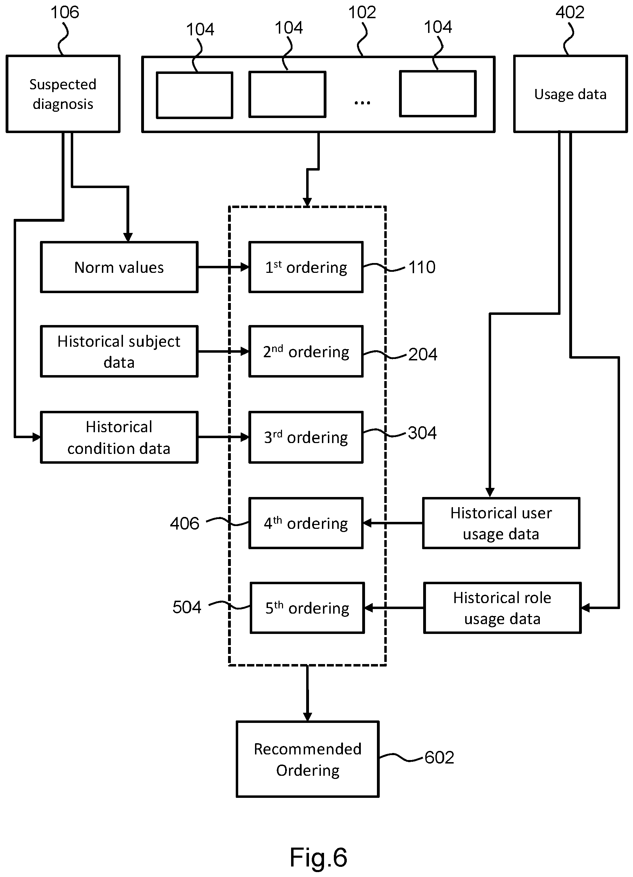

Fig. 6 shows the generation of a recommended ordering 602. Role-specific recommendations, a suspected diagnosis and the point in the treatment trajectory can be used to create insights in decision-making. For example, the point in the trajectory at which a particular data type is crucial or decisive can be determined. These insights in decision making may result in treatment recommendations.

Furthermore, these insights can reveal which of the five orderings 110, 204, 304, 406 and 504 is most valuable/important for making the eventual diagnosis and treatment plan. Thus, the orders of importance can be dynamically weighted over the course of treatment to determine a recommended ordering 602. The recommended ordering 602 can be updated automatically over the course of treatment as the weights are changed based on the insights obtained.

A neural network (e.g., a back-propagation neural network) can be trained based on said insights (e.g., as determined by an expert) and thus determine the weights for the different orders of importance.

It will be appreciated that different orders of importance can be determined from other types of historical data. Similarly, different orders of importance can be generated by combining the different orders of importance as described above.

The skilled person would be readily capable of developing a processor for carrying out any herein described method. Thus, each step of a flow chart may represent a different action performed by a processor and may be performed by a respective module of the processing processor.

One or more steps of any of the methods described herein may be performed by one or more processors. A processor comprises an electronic circuit suitable for processing data. Any method described herein may be computer-implemented, where computer-implemented means that the steps of said methods are performed by one or more computers and where a computer is defined as a device suitable for processing data. A computer may be suitable for processing data according to prescribed instructions.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

Any methods described herein exclude a method for performing mental acts as such.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer-implemented method for ordering health data of a subject to be displayed to a user, the health data comprising subject data for a plurality of data types, the method comprising determining one or more orders of importance for the data types based on historical data for each data type.

2. The method of claim 1, further comprising displaying the subject data, for each data type, based on one or more of the orders of importance.

3. The method of claim 1 or 2, wherein determining one of the orders of importance comprises determining differences between the subject data, for each data type, and historical norm values for each corresponding data type, wherein the ordering of the data types is based on the determined differences.

4. The method of any of claims 1 to 3, wherein determining one of the orders of importance comprises comparing the subject data, for each data type, to historical subject data of the subject, for each corresponding data type, to determine subject-specific differences between the subject data and the historical subject data for each data type, wherein the ordering of the data types is based on the determined subject-specific differences.

5. The method of any of claims 1 to 4, wherein determining one of the orders of importance is based on a condition-specific order for a suspected clinical condition of the subject, wherein the condition-specific order is based on a historical importance of each data type for the suspected clinical condition.

6. The method of any of claims 1 to 5, wherein determining one of the orders of importance is based on a user-specific order for the user requesting the subject data to be displayed, wherein the user-specific order is based on the user's historical preferences for displaying the data types.

7. The method of any of claim 1 to 6, wherein determining one of the orders of importance is based on a role-specific order corresponding to the role of the user requesting the subject data to be displayed, wherein the role-specific order is based on historical preferences for displaying the data types for users of the same role.

8. The method of any of claims 5 to 7, comprising determining a plurality of orders of importance and further comprising comparing the data types available in the health data of the subject to the data types available in the condition-specific, user-specific and/or role-specific orders and identifying missing data types in the health data.

9. The method of any of claims 1 to 8, comprising determining a plurality of orders of importance and further comprising comparing the determined orders of importance to each other and identifying a most relevant order of importance for a suspected clinical condition of the subject.

10. The method of any of claims 1 to 9, further comprising comparing the determined orders of importance to each other and determining a treatment recommendation for the subject.

11. A computer program carrier comprising computer program code which, when executed on a computer, causes the computer to perform all of the steps according to any of claims 1 to 10.

12. A system for ordering health data of a subject to be displayed to a user, the health data comprising subject data for a plurality of data types, the system comprising a processor configured to determine one or more orders of importance for the data types based on historical data for each data type.

13. The system of claim 12, wherein the processor is further configured to display the subject data, for each data type, based on one or more of the orders of importance.

14. The system of claims 12 or 13, wherein the processor is configured to determine a plurality of orders of importance and further configured to compare the determined orders of importance to each other and identifying a most relevant order of importance for a suspected clinical condition of the subject.

15. The system of any of claims 12 to 14, wherein the processor is further configured to compare the determined orders of importance to each other and determining a treatment recommendation for the subject.
